# EUROPEAN PATENT SPECIFICATION

(11) **EP 3 390 060 B1**
(45) Date of publication and mention of the grant of the patent: **18.08.2021**
(21) Application number: 16809786.3
(22) Date of filing: 08.12.2016
(51) Int. Cl.: B41J 3/407, A61Q 1/02, A61Q 1/04, A61Q 1/08, A61K 8/02, B41M 3/00

(54) **PROCESS FOR COLOURING A BASE COSMETIC COMPOSITION**
VERFAHREN ZUR FÄRBUNG EINER KOSMETISCHEN BASISZUSAMMENSETZUNG
PROCÉDÉ DE COLORATION D'UNE COMPOSITION COSMÉTIQUE DE BASE

(30) Priority: 18.12.2015 FR 1562877
(43) Date of publication of application: 24.10.2018
(73) Proprietor: L'Oréal, 75008 Paris (FR)
(72) Inventor: GIRON, Franck, 77400 Lagny sur Marne (FR); SAMAIN, Henri, 91570 Bièvres (FR)
(74) Representative: Nony
(86) International application number: PCT/EP2016/080338
(87) International publication number: WO 2017/102562

(56) References cited:
- WO-A1-2015/097620
- WO-A1-2015/168524
- FR-A1- 2 759 941
- TechCrunch: "Print Your Own Makeup With Mink | Disrupt NY 2014", , 5 May 2014 (2014-05-05), page 1 pp., XP054976603, Retrieved from the Internet: URL:https://www.youtube.com/watch?v=cBZHFU QiP8Q [retrieved on 2016-06-17]

## Description

The present invention relates to the personalized colouration of a cosmetic composition by deposition, by printing, of at least one ink onto the composition.

### Prior art and technical problem

It is known to produce cosmetic compositions of different colours then to combine them in the same case. This solution has several drawbacks. Firstly, as it is difficult to produce cosmetic compositions in compact powder form of small surface area, the combination thereof generates bulky assemblies, or then the number of cosmetic compositions is reduced, cutting down, however, the choice of colours. Furthermore, this solution is too difficult to implement to be able to be carried out at home. Finally, this does not enable the user to vary or select the surfaces that he or she wishes to assign to each colour.

International application WO 2015/168524 describes a process consisting in taking a basic cosmetic composition, and, by means of an inkjet printer, covering it with a colouring composition. The printing is carried out over the entire surface of the basic composition.

WO2015/168524 discloses a device for producing a cosmetic composition being in the form of a printer that is modified to receive and process cosmetic components. The device includes a printer housing that includes at least one print head and an opening for receiving a substrate and allow positioning of the substrate in relation to the at least one print head. At least one replaceable cartridge is provided and contains a cosmetic substance. The at least one replaceable cartridge is operatively coupled to the at least one print head such that the cosmetic substance can be applied to the substrate through the at least one print head. The resulting cosmetic composition is made of at least one cosmetic substance and the cosmetic composition is a transferable material that can be removed from the substrate and applied to a part of a human body. Youtube video TechCrunch « Print your own makeup with Mink" (XP054976603) discloses some aspects of a similar device.

WO2015/097620 relates to a makeup device comprising a substrate defining a printing surface having at least one region printed with at least one coloring dye layer to be applied onto human keratinous material. Said at least one coloring dye has been deposited, via printing, onto the printing surface by at least one printer, particularly a digital printer, is not covered by an adhesive; and produces, after application onto the keratinous material, at least one visible optical effect, namely color and/or sheen.

In FR 2 759 941, the decoration of the surface of cosmetic powders or creams uses an ink-jet printer delivering pigments and/or colourants that are safe to the skin. The ink is on an aqueous base. The cosmetic product may be a compacted powder, a paste, such as lipstick, a gel or an emulsion. The ink-jet printer uses four nozzles connected to ink cartridges containing respectively yellow, red, blue and black ink. These colours are combined to form designs of any colour on the surface of the cosmetic.

### Objectives of the invention

There is thus a need to be able to easily produce compositions, in particular in compact powder form, with several coloured zones of small surface area. In particular, there is a need for such compositions to be obtained without requiring the assembly of various cosmetic compositions.

### Process

One subject of the present invention is thus, according to one of its aspects, a process for colouring a base cosmetic composition, comprising the step consisting in producing, on at least one first zone and one second zone, which are different, at the surface of said composition, two printings of at least one ink originating from an inkjet printer, the base composition being a pulverulent in compact powder form, the at least one ink being an aqueous ink comprising a weight concentration of at least 50% of water and having a viscosity ranging from 1 mPa.s to 500 mPa.s at 25°C, the base composition and the ink being such that the time needed for the complete penetration, at ambient temperature of 20°C and under atmospheric pressure, into the base composition of an amount of 20 µl of water deposited at its surface is less than 30 s and such that the time needed for the complete penetration into the base composition of the same amount, 20 µl, of ink deposited at its surface is less than 15 s, wherein the ink is deposited in a sufficient amount so as to diffuse through a depth of the base composition greater than or equal to 1 mm, for each zone.

By means of the invention, it is possible to produce a cosmetic composition printed with two different zones, in particular that are close or even juxtaposed, the printings of which have relatively sharp borders as seen by the user. A rapid vertical diffusion of the ink, that is to say in the depth direction, makes it possible to minimize the lateral diffusion capable of making the contour of the printing blurred and without the required sharpness. If the borders between the zones are not sufficiently sharp, the area of intersection at the border between two adjacent zones is lost and unusable for the user. This border problem is particularly critical if it is desired to produce zones of small surface areas.

The base composition and the ink may in particular be such that the time needed for the complete penetration into the base composition of an amount of 20 µl of water deposited at its surface is less than 12 s and such that the time needed for the complete penetration into the base composition of the same amount, 20 µl, of ink deposited at its surface is less than 12 s.

The invention makes it possible to have a cosmetic composition in compact powder form, having at least two separate zones coloured in a personalized manner, the two zones being preferably coloured with different colours.

The coloured zones preferably have a surface area of at least 0.5 cm² in order that the user can withdraw the composition in the zone of his or her choice. The printed cosmetic composition according to the invention has at least two zones that enable the user to have at least two colours in order to achieve and make a success of his/her makeup result.

The ink is deposited in a sufficient amount so as to diffuse through a depth of the base composition greater than or equal to 1 mm, for each zone. This makes it possible to colour the base composition over a sufficient depth in order to be able to withdraw an amount thereof that is compatible with the achievement of one or more makeup results.

The step of producing two printings of ink(s) may be carried out so that the distance d between the adjacent edges of the depositions of the two zones is less than or equal to the sum (l₁ + l₂) of the lateral diffusion widths of each of the inks multiplied by a coefficient k between 1 and 2, i.e. d ≤ (l₁+l₂)*k.

The lateral diffusion width (l₁, l₂) is preferably less than or equal to 5 mm, better still less than or equal to 2 mm, and even better still less than or equal to 1 mm.

In one particular embodiment, an ink of a first colour is deposited on the first zone and an ink of a second colour is deposited on the second zone.

The number of zones may be greater than two, the number of colours of inks deposited by printing may be equal to two, the zones being positioned together in a predetermined manner, especially in alternation, in particular in check pattern form.

As a variant, the number of zones and of colours of inks deposited is greater than two.

Each zone of the base composition may be delimited by a demarcation element different from the composition. Each zone may be defined by a small dish in which the composition in this zone is housed, the small dish of one zone being separate from that of an adjacent zone.

As a variant, at least two zones are defined by a same small dish containing the composition.

Each zone may have a polygonal or non-polygonal contour, in particular a circular or non-circular, for example oval, contour. It is thus possible to produce various patterns on the base composition.

The deposition of ink(s) is advantageously carried out over the whole of the surface of the base composition.

### Base composition

The base composition may be hydrophobic or hydrophilic.

According to a first aspect of the invention, the base composition is hydrophobic.

The expression "hydrophobic base composition" is understood to mean a composition that repels water. In particular, a hydrophobic base composition is not soluble in water or in polar compositions or is very sparingly soluble in water or in polar compositions. In other words, a hydrophobic base composition does not create hydrogen bonds with the water molecules. Thus, a hydrophobic base composition comprises for the most part hydrophobic compounds.

In particular, a hydrophobic base composition is formed entirely or partly of a fatty phase.

The fatty phase of a composition of the invention may in particular comprise at least one fatty substance that is liquid at ambient temperature and/or a fatty substance that is solid at ambient temperature, such as waxes, butters, pasty fatty substances and gums, and mixtures thereof.

The fatty phase of the composition according to the invention may especially comprise, as liquid fatty substance, at least one volatile or non-volatile oil or a mixture thereof. These volatile or non-volatile oils may for example be hydrocarbon-based oils or silicone oils, or mixtures thereof.

According to a second aspect of the invention, the base composition is hydrophilic.

The expression "hydrophilic base composition" is understood to mean a composition that has an affinity for water. In particular, a hydrophilic base composition is soluble in water or in polar solvents. In other words, a hydrophilic base composition has the ability to create hydrogen bonds with water or polar solvents.

Thus, a hydrophilic base composition comprises for the most part hydrophilic compounds.

In particular, a hydrophilic base composition is formed entirely or partly of an aqueous phase.

The aqueous phase may comprise water. It may also comprise at least one water-soluble solvent and/or at least one water-soluble film-forming polymer.

The water-soluble solvents that can be used in the compositions according to the invention may also be volatile. Among the water-soluble solvents that may be used in the compositions in accordance with the invention, mention may be made in particular of lower monoalcohols containing from 1 to 5 carbon atoms such as ethanol and isopropanol, and glycols containing from 2 to 8 carbon atoms such as ethylene glycol, propylene glycol, 1,3-butylene glycol and dipropylene glycol.

Generally, the base compositions according to the invention may contain fillers or pigments that are coated such as talc, sericite, gum beads, titanium dioxide, zinc oxide, aluminium, in particular coated with PDMS, methylhydropolysiloxane, hydrogenated palm oil, lauroyl lysine, silsesquioxane resin, and for example the references CI:77019, CI: 77891, KSP100 from Shin Etsu, and SA-TA-13R from Shiyomi Kasei, and mixtures thereof.

They may also comprise fillers, pigments or particles of polymers that are not coated such as perlite, iron oxides, talc, aerogels, PMMA, nylon, and mixtures thereof.

The base composition is pulverulent in compact powder form.

The base composition may comprise any customary compound used in pulverulent cosmetic compositions.

For obvious reasons, the choice of the nature and amount of these compositions is clearly within the competence of those skilled in the art.

The term "compact powder" is intended to mean a mass of product of which the cohesion is at least partly provided by compacting or pressing during the manufacture. In particular, by taking a measurement using a TA.XT.plus Texture Analyser texturometer sold by the company Stable Micro Systems, the compact powder according to the invention may advantageously have a compressive strength of between 0.1 and 2.5 kg and especially between 0.2 and 1.0 kg, relative to the surface area of the spindle used (in the present case 7.07 mm²). The measurement of this strength is performed by moving an SMS P/3 flat-headed cylindrical spindle over a distance of 1.5 mm and at a speed of 0.5 mm/second in contact with the powder.

The base composition may be white before deposition of the printings of ink(s).

The base composition is, for example, a foundation, a blusher, an eyeshadow or a lipstick.

### Inks

The ink(s) deposited on the base composition, by printing, is (are) one or more aqueous inks, comprising a weight concentration of at least 50% of water. The ink(s) can comprise an additional hydrophilic solvent chosen from alcohols, such as lower monoalcohols having from 1 to 5 carbon atoms such as ethanol and isopropanol, glycols having from 2 to 8 carbon atoms, such as ethylene glycol, propylene glycol, 1,3-butylene glycol and dipropylene glycol, C3 and C4 ketones, in particular acetone, and C2-C4 aldehydes and polyols, such as for example chosen from ethylene glycol, pentaerythritol, trimethylolpropane, propylene glycol, 1,3-propanediol, butylene glycol, isoprene glycol, pentylene glycol, hexylene glycol, glycerol, polyglycerols, such as glycerol oligomers, for instance diglycerol, polyethylene glycols, and mixtures thereof.

The ink(s) deposited on the base composition, by printing, is (are) advantageously offset inks, inks for flexography and photogravure, containing alcohols such as propanol, ethanol or butanol, glycol esters, alkyl acetates, ketones such as acetone or diacetone alcohol, or mixtures thereof.

The total amount of ink(s) deposited on the base composition is advantageously greater than or equal to 0.2 mg/cm².

The or each ink comprises a colourant. The colourant may be present in the ink in a weight content ranging from 0.01% to 60%, preferably ranging from 0.1% to 40%, or even from 0.1% to 30% and preferentially ranging from 0.5% to 20%, relative to the total weight of the ink.

The ink may comprise one or more colourants chosen from water-soluble dyes and liposoluble dyes.

Among the water-soluble dyes, mention may be made of the disodium salt of ponceau, the disodium salt of alizarin green, quinoline yellow, the trisodium salt of amaranth, the disodium salt of tartrazine, the monosodium salt of rhodamine, the disodium salt of fuchsin, xanthophyll and methylene blue.

Among the liposoluble dyes, mention may be made of Sudan Red III (CTFA: D&C Red 17), lutein, quinizarine green (CTFA: D&C Green 6), alizurol purple SS (CTFA: D&C Violet 2), Sudan Brown, D&C Yellow 11, D&C Orange 5, quinoline yellow, curcumin, and carotenoid derivatives such as lycopene, beta-carotene, bixin or capsanthin, and mixtures thereof. The colouring polymers are generally copolymers based on at least two different monomers, at least one of which is a monomeric organic dye. Such polymeric dyes are known to those skilled in the art. Reference may be made, for example, to the following documents: US-5 032 670; US-4 999 418; US-5 106 942; US-5 030 708; US-5 102 980; US-5 043,376; US-5 104 913; US-5 281 659, US-5 194 463; US-4 804 719; WO 92/07913, or else EP 1 048 282.

The ink according to the invention constitutes a cosmetically acceptable medium, i.e. a medium that is compatible with keratin materials such as the skin of the face or the body, the lips, the hair, the eyelashes, the eyebrows and the nails.

The ink is liquid at the time of printing and has a viscosity ranging from 1 mPa.s to 500 mPa.s at 25°C and preferably from 1 mPa.s to 300 mPa.s at 25°C.

The viscosity of an ink according to the invention may be measured according to any process known to those skilled in the art, and in particular according to the following conventional process. At 25°C using a Rheomat 180 viscometer, equipped with a spindle rotating at 200 rpm, those skilled in the art choose the spindle for measuring the viscosity from the spindles M1, M2, M3 and M4 on the basis of their general knowledge, so as to be able to perform the measurement.

The ink may be in emulsion form.

The ink may be chosen from those that are sold for the Gatocopy food-grade printer, in particular of reference A426.

### Third compound

The step of depositing printings of ink(s) may be performed so as to delimit a plurality of zones, the base composition on at least one zone containing or being intended to contain a third compound. Still within this case, the process may comprise the step consisting in depositing, using the printer, said third compound, simultaneously or after the step of depositing printings of ink(s).

The third compound may be chosen from the list made up of cosmetic and/or dermatological active agents.

As active agents that are suitable for the present invention, mention may be made in particular of:
- anti-ageing/anti-wrinkle agents,
- moisturizers (or humectants),
- fragrances,
- neutralizers,
- emollients,
- binders, in particular water-soluble polymers or polymers in latex form,
- free-radical scavengers,
- coalescence agents,
- vitamins,
- screening agents, in particular sunscreens,
- anti-pollution agents and/or free-radical scavengers,
- agents acting on the microcirculation,
- agents acting on the energy metabolism of cells,
- hyaluronic acid,
- anti-glycation agents,
- NO-synthase inhibitors,
- agents for stimulating the synthesis of dermal or epidermal macromolecules and/or for preventing their degradation,
- agents for stimulating fibroblast and/or keratinocyte proliferation or for stimulating keratinocyte differentiation,
- muscle relaxants (dermo-relaxing agents and/or dermo-decontracting agents),
- tensioning agents,
- desquamating agents,
- depigmenting or propigmenting agents,
- anti-acne, and especially anti-seborrhoea and anti-*P*. *acnes* active agents,
- antioxidants and free-radical scavengers,
- saccharides,
- oligosaccharides, polysaccharides, which are hydrolysed or non-hydrolysed, and modified or unmodified,
- amino acids, oligopeptides, peptides, proteins which are hydrolysed or non-hydrolysed, and modified or unmodified, poly amino acids, enzymes,
- animal, plant or mineral waxes,
- ceramides and pseudoceramides,
- hydroxylated organic acids,
- soluble or dispersed anionic polymers,
- soluble or dispersed non-ionic polymers,
- calmatives, and
- mixtures thereof.

The third compound may in particular comprise at least one compound chosen from fragrances, UV-screening agents, moisturizers, binders, anti-acne active agents, and mixtures thereof, and preferably chosen from UV-screening agents and anti-acne active agents.

The amount of cosmetic and/or dermatological active agent(s) obviously depends on the nature of the active agent and on the desired effect, but said active agent(s) generally represent(s) from 0.1% to 40% by weight, relative to the total weight of the composition.

In the case where the third compound is a fragrance, the fragrance may be water-soluble or hydrophobic and peptized.

By way of UV-screening agent, mention may in particular be made of those chosen from water-soluble UV-screening agents, liposoluble UV-screening agents, insoluble UV-screening agents, and mixtures thereof. Among these UV-screening agents, a distinction can be made between water-soluble organic screening agents, liposoluble organic screening agents, insoluble organic screening agents and inorganic screening agents.

Preferably, it is a water-soluble UV-screening agent or an inorganic UV-screening agent.

Among the water-soluble organic UVA-screening agents that can be used according to the present invention, mention may be made of benzene-1,4-di(3-methylidene-10-camphorsulfonic acid) (INCI name: Terephthalylidene Dicamphor Sulfonic Acid) and the various salts thereof, described in particular in patent applications FR 2 528 420 and FR 2 639 347, compounds comprising at least two benzoazolyl groups comprising sulfonic groups, such as those described in patent application EP 0 669 323, and benzophenone compounds comprising at least one sulfonic acid function.

The water-soluble organic UVB-screening agents are in particular chosen from water-soluble cinnamic derivatives, such as ferulic acid or 3-methoxy-4-hydroxycinnamic acid; water-soluble benzylidenecamphor compounds; water-soluble phenylbenzimidazole compounds; water-soluble p-aminobenzoic (PABA) compounds and water-soluble salicylic compounds.

The inorganic UV-screening agents used in accordance with the present invention are metal oxide pigments. They may be chosen in particular from titanium oxide, zinc oxide, iron oxide, zirconium oxide and cerium oxide, or mixtures thereof.

The term "moisturizer" or "humectant" is intended to mean:
- either a compound which acts on the barrier function, for the purpose of maintaining the hydration of the stratum corneum, or an occlusive compound. Mention may be made of ceramides, sphingoid-based compounds, lecithins, glycosphingolipids, phospholipids, cholesterol and derivatives thereof, phytosterols (stigmasterol, β-sitosterol, campesterol), essential fatty acids, 1,2-diacylglycerol, 4-chromanone, pentacyclic triterpenes such as ursolic acid, petroleum jelly and lanolin;
- or a compound which directly increases the water content of the stratum corneum, such as threalose and derivatives thereof, glycerol, hyaluronic acid and derivatives thereof, glycerol, pentanediol, sodium pidolate, serine, xylitol, sodium lactate, glyceryl polyacrylate, ectoin and derivatives thereof, chitosan, oligosaccharides and polysaccharides, cyclic carbonates, N-lauroylpyrrolidonecarboxylic acid, and N-α-benzoyl-L-arginine;
- or a compound which activates the sebaceous glands, such as steroidal derivatives (including DHEA, 7-oxidized and/or 17-alkylated derivatives thereof and sapogenins), methyl dihydrojasmonate, and vitamin D and derivatives thereof.

In the case where the third compound is a water-soluble polymer, the latter can be chosen from the group made up of an acrylate copolymer, a methacrylate, a polyester and a polyurethane.

The term "anti-acne active agent" is especially understood to mean any active agent that has effects on the specific flora of oily skin, for instance *Propionibacterium acnes* (*P. acnes*). These effects may be bactericidal.

Antibactericidal active agents that may especially be mentioned include:
- the active agents and preserving agents with antimicrobial activity cited in DE 1032467,
- asiatic acid,
- the monoethanolamine salt of 1-hydroxy-4-methyl-6-trimethylpentyl-2-pyridone (INCI name: piroctone olamine),
- citronellic acid, perillic acid (or 4-isopropenylcyclohex-1-enecarboxylic acid),
- 2-ethylhexyl glycerol ether (INCI name: ethylhexylglycerin),
- glyceryl caprylate/caprate,
- calcium sodium phosphosilicate,
- silver-based particles,
- hop cone (*Humulus lupulus*) extract obtained by supercritical CO₂ extraction,
- St Johns wort extract obtained by supercritical CO₂ extraction,
- the mixture of extracts of roots of *Scutellaria baicalensis, Paeonia suffruticosa* and *Glycyrrhiza glabra,*
- argan tree extract,
- extracts of bearberry leaves,
- 10-hydroxy-2-decanoic acid, sodium ursolate, diiodomethyl p-tolyl sulfone, malachite powder, zinc oxide, octadecenedioic acid; ellagic acid; 2,4,4'-trichloro-2'-hydroxydiphenyl ether (or triclosan), 1-(3',4'-dichlorophenyl)-3-(4'-chlorophenyl)urea (or triclocarban), 3,4,4'-trichlorocarbanilide, 3',4',5'-trichlorosalicylanilide, phenoxyethanol, phenoxypropanol, phenoxyisopropanol, hexamidine isethionate, metronidazole and salts thereof, miconazole and salts thereof, itraconazole, terconazole, econazole, ketoconazole, saperconazole, fluconazole, clotrimazole, butoconazole, oxiconazole, sulfaconazole, sulconazole, terbinafine, ciclopirox, ciclopiroxolamine, undecylenic acid and salts thereof, benzoyl peroxide, 3-hydroxybenzoic acid, 4-hydroxybenzoic acid, phytic acid, N-acetyl-L-cysteine, lipoic acid, azelaic acid and salts thereof, arachidonic acid, resorcinol, octoxyglycerin or octoglycerin, octanoylglycine, caprylyl glycol, dichlorophenylimidazoldioxolane and derivatives thereof described in patent application WO 93/18743, iodopropynyl butylcarbamate, 3,7,11-trimethyldodeca-2,5,10-trienol or farnesol, phytosphingosines; quaternary ammonium salts, for instance cetyltrimethylammonium salts and cetylpyridinium salts, and
- mixtures thereof.

Mention may also be made of certain surfactants with an antimicrobial effect, for instance sodium cocoamphoacetate or disodium cocoamphodiacetate, betaines, sodium lauryl ether sulfate, decyl glucoside, malates of branched C₁₂-₁₃ dialcohols, propylene glycol monoesters, for instance propylene glycol monolaurate, monocaprylate or monocaprate, lauryl dimethylamine betaine, and also polyquaternary ammoniums such as those described in patent FR 0 108 283, and mixtures thereof.

Other additional anti-acne active agents may be added to the abovementioned anti-acne active agents. Mention may especially be made of active agents having effects that act against bacterial adhesion or else active agents that act on the biofilm of bacteria to prevent them from multiplying.

As agents that prevent and/or reduce the adhesion of the microorganisms, mention may especially be made of: phytantriol and derivatives thereof as described in patent application EP 1 529 523, plant oils such as wheatgerm oil, calendula oil, castor oil, olive oil, avocado oil, sweet almond oil, groundnut oil, jojoba oil, sesame seed oil, apricot kernel oil, sunflower oil and macadamia oil, described in patent EP 1 133 979, or else certain surfactants such as disodium cocoamphodiacetate, oxyethylenated (7 EO) glyceryl cocoate, hexadecenyl succinate 18, octoxyglyceryl palmitate, octoxyglyceryl behenate, dioctyl adipate, PPG-15 stearyl ether, and the tartrate of branched C₁₂-C₁₃ dialcohols described in patent EP 1 129 694, and mixtures thereof. In particular with regard to the propagation of *P. acnes,* or as active agents that act on the biofilm of bacteria to prevent them from proliferating, mention may be made of pentylene glycol, Nylon-6,6 (polyamide 6,6 fibres), rice bran oil, polyvinyl alcohol such as Celvol 540 PV alcohol® from Celanese Chemical, rapeseed oil such as Akorex L® from Karlshamns, and fructose derivatives, and mixtures thereof.

### Inkjet printer

The printer used is of digital type. The term "digital printer" is intended to mean a machine capable of printing in the form of pixels using digital data, different from a machine comprising a printing form. The printer is an inkjet printer, for example a thermal or piezoelectric inkjet printer.

The printer may be a food-grade inkjet printer of the Gatocopy A426 type allowing printing onto non-flat objects.

The step of depositing printings of ink(s) may be carried out so as to produce a gradation of colours between two adjacent zones.

The step of depositing printings of ink(s) may consist in carrying out a number n of printing passes, n being greater than 1, n preferably being between 5 and 200. The larger the number of printing passes, the greater the amount of ink deposited. The higher the number of printing passes, the greater the depth of diffusion into the composition.

Each printing is for example a multicolour printing, in particular being carried out as three-colour printing or four-colour printing, or printing with more than four inks.

The printing may use several different inks, especially inks of different colours. The printings produced may be multicolour printings, in particular being carried out as three-colour printing or four-colour printing, or printing with more than four inks.

The printing may use only colouring inks corresponding to primary colours. As a variant, the printing uses both colouring inks corresponding to primary colours and at least one ink corresponding to a non-primary colour. In one variant, the printing may use black and/or white colouring inks.

### Choice and storage of the printing

The colour or colours printed by deposition of the printings of ink(s) may be stored in an electronic memory. Thus, the mere recall of the memory is sufficient to again implement the process with the same colour(s) of ink(s).

Each printing may be carried out according to colour information, entered by the user, in particular selected on a screen, or measured, in particular on the skin and/or according to a target colour to be achieved during the application. The process may comprise a step of selecting the colour(s) of each printing according to the colour of the keratin materials to be made up, and in particular an acquisition by a camera of the colour of said keratin materials and/or according to target colour(s) to be achieved during the application.

### Figures

The invention may be better understood with the following reading of nonlimiting implementation examples thereof, and with examination of the attached drawing, in which:
- Figure 1 is a schematic view of an example of a cosmetic composition produced according to the process in accordance with the invention,
- Figure 2 is a photograph illustrating the measurement of the lateral diffusion of the ink around the printing zone,
- Figure 3 is a photograph partially illustrating a cross section of the base composition after printing according to the process in accordance with the invention,
- Figures 4 to 6 are photographs illustrating, in perspective and in cross section, various base compositions after printing according to the process in accordance with the invention.

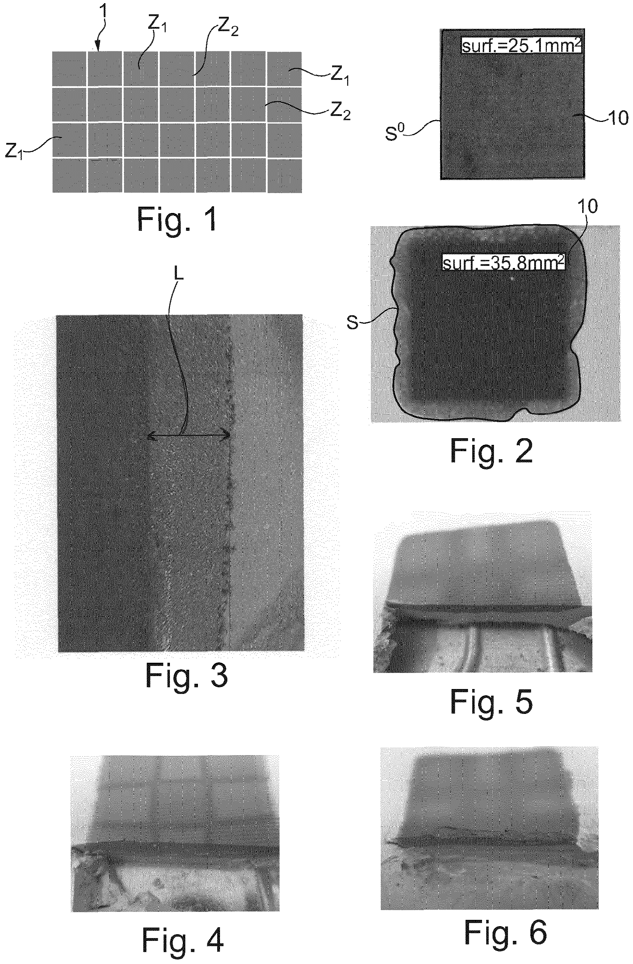

Figure 1 shows an example of a base composition 1 after implementation of the process according to the invention.

In this example, the printing on two different zones of the base composition 1, namely zones Z1, of square shape in this example, and zones Z2 in the form of lines together forming a grid pattern that separates the zones Z1 from one another, is carried out several times. The printing carried out on the zones Z1 enables the deposition of an ink of non-white colour. The printing carried out on the zones Z2 enables the deposition of another, more neutral colour.

The base composition 1 may comprise a third compound in the form of a dermatological active agent, so that the composition obtained makes it possible to act as a guide for the application of said dermatological active agent.

As a variant, the process comprises the step of printing, onto the base composition, of this third compound, for example contained in a fluid composition, in similar or different amounts between the various zones Z1. This printing step may be at the same time as the ink printing step or afterwards.

### Tests

### Diffusion width, distance between two zones

The evaluation of the lateral diffusion outside of the deposition of ink at the moment of printing is carried out in the following manner:
50 printing passes are carried out on a 5 × 5 mm square of a base composition 10. The colour of the ink is chosen so as to stand out from the colour of the base composition.

The printing takes place under controlled temperature and humidity conditions (20°C, 50% RH).

Then, afterwards, it is left to dry for 24 hours. This drying time is important. Indeed, formulae that give sharp edges just after printing may become blurred after 24 h.

Next, a photograph is taken with the HiROX KH 8700 video microscope equipped with the MX-macro Z VI lens. This photograph is visible in Figure 2.

Using image analysis software, the outline of the pattern obtained after lateral diffusion of all the edges of the printing is plotted, taking care to define an object with a closed outline. The software gives the surface area S of the object, S being 35.8 mm² in this example, seen in the photograph from the bottom of Figure 2.

1 printing pass is carried out on a 5 × 5 mm square under the same conditions as above. The reference surface area with no diffusion, S°, of 25.1 mm² is obtained. The photograph is visible at the top of Figure 2.

Then, the lateral diffusion D1 all around the initial printing zone is determined by the following formula: Dl= (S-S°)/20.

### Measurement of the depth of diffusion of ink(s)

After carrying out the printings of the process according to the invention, the cosmetic composition impregnated with ink(s) is left to dry for 24 hours. A transverse section is cut in the block of the cosmetic composition using a knife. One of the two halves is freed and a photograph of the slice is taken. It should be noted that it is not possible to measure exactly by means of the slice because of the presence of the small dish. Thus, a photograph, visible in Figure 3, is taken, with an angle α, in this example equal to 45°, which results in the thickness measured being corrected by a factor 1/cos(α), i.e. *2* if the angle is 45°.

By virtue of a calibration chart and image analysis software, the depth L of penetration of the ink(s), and therefore the thickness L of the layer impregnated with ink(s), are extracted therefrom.

The work is carried out with the HiROX KH 8700 video microscope equipped with the MX-macro Z VI lens and using proprietary software.

### Rate of penetration of the ink

The test to be carried out in order to determine the rate of penetration of the ink into the base composition is the following:
1) A 20 µl drop of water is applied to the surface of the compact (by means of a micropipette). Next, the penetration of the drop is monitored and the time that it takes to disappear is noted. In order to do this, the deposition and the penetration of the drop are filmed from a grazing direction of observation until the disappearance of the drop. This disappearance is observed when liquid is no longer seen at the surface giving rise to either the disappearance of the brightness, or the stabilization of the aureole and the duration of the sequence between the deposition of the drop and its disappearance is marked off a posteriori on the video. This time is referred to as td1.
2) The same test is carried out with the ink instead of the water. This time is referred to as td2.

The base compositions that fall within the context of the invention are those which, with the ink considered, have:
- td1 < 30 s and td2 < 15 s and
- preferably: td1 < 12 s and td2 < 12 s.

### Examples

Use is made, in these examples, of the following two inks (manufacturer = Lesepidado):
- Epson EU/ME Magenta ink (ref: ART.76304000)
- Epson EU/ME Cyan ink (ref: ART.76304002)

Several base compositions in the form of commercial compact powders, also referred to as compacts, are taken and the penetration rate test described above is carried out in order to measure the times td1 and td2 for each of the compositions.

Next, a blue and magenta check pattern is printed with the two inks above formed of nine squares of 5 mm × 5 mm per square. The printing is carried out by performing 50 printing passes with the Gatocopy printer.

There is a waiting period of 24 hours for the pattern to stabilize. Then, the reproduction and sharpness quality of the check pattern is evaluated visually. For this, a panel of 4 people is used, in order to give a score ranging from 0 to 5.

Next, the depth of diffusion and the printing quality at depth are evaluated. The evaluation is made in mm.

| Compacts | | td2 (s) | td1 (s) | Sharpness | Depth (mm) |
|---|---|---|---|---|---|
| Lancôme Absolue Compact | 1 | 5 | 5 | 3 | 2 |
| L'Oréal Paris True Match Mineral | 1 | 3 | 3 | 3 | 2 |
| Lancôme Belle de teint | 1 | 6 | 9 | 3 | 2 |
| L'Oréal Paris FAP_Matte106 | 2 | 1 | 2 | 3 | 1.75 |
| La Roche Posay Toleriane Mineral | 3 | 10 | >60 | 2 | 1 |
| Lancôme Transluscence Powder | 3 | 5 | >60 | 2 | 1 |
| L'Oréal Paris Perfect Match | 4 | 3 | >60 | 1 | 1 |
| L'Oréal Paris Infallible | 4 | 11 | >60 | 2 | 1.5 |
| Moisturizing complexion | 5 | 17 | 4 | 0 | 1.5 |

Only the printings that achieve a sharpness score of 3 are satisfactory. Furthermore, it is seen that these formulae give the best deep penetrations into the base composition.

The results obtained for the compositions entitled True Match Mineral, Infallible and Perfect Match are illustrated respectively in Figures 4, 5 and 6. In the case of Figure 4, a sharpness of 3 is visualized, which is therefore satisfactory, after implementation of the process according to the invention on the composition sold under the name True Match Mineral. In the case of Figure 5, a sharpness of 2 is visualized, which is insufficient, after implementation of the process according to the invention on the composition sold under the name Infallible. Finally, in the case of Figure 6, a sharpness of 1 is visualized, which is insufficient, after implementation of the process according to the invention on the composition sold under the name Perfect Match.

The depth of diffusion in the True Match Mineral composition visible in the cross section produced and visible in Figure 4 is greater than that of the other two compositions illustrated in Figures 5 and 6.

## Claims

1. Process for colouring a base cosmetic composition, comprising the step consisting in producing, on at least one first zone and one second zone, which are different, at the surface of said composition, two printings of at least one ink originating from an inkjet printer, the base composition being a pulverulent in compact powder form, the at least one ink being an aqueous ink comprising a weight concentration of at least 50% of water and having a viscosity ranging from 1 mPa.s to 500 mPa.s at 25°C, the base composition and the ink being such that the time needed for the complete penetration, at ambient temperature of 20°C and under atmospheric pressure, into the base composition of an amount of 20 µl of water deposited at its surface is less than 30 s and such that the time needed for the complete penetration into the base composition of the same amount, 20 µl, of ink deposited at its surface is less than 15 s, wherein the ink is deposited in a sufficient amount so as to diffuse through a depth of the base composition greater than or equal to 1 mm, for each zone.

2. Process according to Claim 1, in which the base composition and the ink are such that the time needed for the complete penetration into the base composition of an amount of 20 µl of water deposited at its surface is less than 12 s and such that the time needed for the complete penetration into the base composition of the same amount, 20 µl, of ink deposited at its surface is less than 12 s.

3. Process according to Claim 1, the step of producing two printings of ink(s) being carried out so that the distance d between the adjacent edges of the depositions of the two zones is less than or equal to the sum (l₁ + l₂) of the lateral diffusion widths of each of the inks multiplied by a coefficient k between 1 and 2, i.e. d ≤ (l₁+l₂)*k, the lateral diffusion width (l₁, l₂) being preferably less than or equal to 5 mm, preferably less than or equal to 2 mm, preferably less than or equal to 1 mm.

4. Process according to any one of the preceding claims, an ink of a first colour being deposited on the first zone and an ink of a second colour being deposited on the second zone.

5. Process according to any one of the preceding claims, the number of zones being greater than two, the number of colours of inks deposited by printing being equal to two, the zones being positioned together in a predetermined manner, especially in alternation, in particular in check pattern form, or the number of zones and of colours of inks deposited being greater than two.

6. Process according to any one of the preceding claims, each zone of the base composition being delimited by a demarcation element different from the composition, in particular each zone being defined by a small dish in which the composition in this zone is housed, the small dish of this zone being separate from that of an adjacent zone or at least two zones being defined by a same small dish containing the composition.

7. Process according to any one of the preceding claims, each zone having a polygonal or non-polygonal contour, in particular a circular or non-circular, for example oval, contour.

8. Process according to any one of the preceding claims, the step of depositing printings of ink(s) being carried out so as to produce a gradation of colours between two adjacent zones.

9. Process according to any one of the preceding claims, the step of depositing printings of ink(s) being performed so as to delimit a plurality of zones, the base composition on at least one zone containing or being intended to contain a third compound, the third compound being preferably an active agent chosen from the group made up of cosmetic and/or dermatological active agents, such as UV-screening agents and anti-acne active agents, the process comprising preferably the step consisting in depositing, using the printer, said third compound, simultaneously or after the step of depositing printings of ink(s).

10. Process according to any one of the preceding claims, the step of depositing printings of ink(s) consisting in carrying out a number *n*>*1* of printing passes, *n* preferably being between 5 and 200.

11. Process according to any one of the preceding claims, the base composition being white before deposition of the printings of ink(s), the base composition being preferably a foundation, a blusher, an eyeshadow or a lipstick.

12. Process according to any one of the preceding claims, the deposition of ink(s) over all of the zones covering the whole of the surface of the base composition.

13. Process according to any one of the preceding claims, the total amount of ink(s) deposited on the base composition being greater than or equal to 0.2 mg/cm².

## Patentansprüche

1. Verfahren zum Färben einer kosmetischen Basiszusammensetzung, das den Schritt umfasst, der darin besteht, auf mindestens einer ersten Zone und einer zweiten Zone, die unterschiedlich sind, auf der Oberfläche der Zusammensetzung zwei Drucke mindestens einer Tinte, die aus einem Tintenstrahldrucker stammt, herzustellen, wobei die Basiszusammensetzung ein Pulver in kompakter Pulverform ist, wobei die mindestens eine Tinte eine wässrige Tinte ist, die eine Gewichtskonzentration von mindestens 50 % Wasser umfasst und eine Viskosität im Bereich von 1 mPa.s bis 500 mPa.s bei 25 °C aufweist, wobei die Basiszusammensetzung und die Tinte so beschaffen sind, dass die Zeit, die benötigt wird für das vollständige Eindringen bei Umgebungstemperatur von 20°C und unter atmosphärischem Druck in die Basiszusammensetzung einer Menge von 20 µl Wasser, das an ihrer Oberfläche aufgebracht wird, weniger als 30 s beträgt und so, dass die Zeit, die benötigt wird für das vollständige Eindringen in die Basiszusammensetzung der gleichen Menge, 20 µl, an Tinte, die an ihrer Oberfläche aufgebracht wird, weniger als 15 s beträgt, wobei die Tinte in einer ausreichenden Menge für jede Zone aufgebracht wird, um durch eine Tiefe der Basiszusammensetzung, die größer als oder gleich 1 mm ist, zu diffundieren.

2. Verfahren nach Anspruch 1, bei dem die Basiszusammensetzung und die Tinte so beschaffen sind, dass die Zeit, die benötigt wird für das vollständige Eindringen in die Basiszusammensetzung einer Menge von 20 µl Wasser, das an ihrer Oberfläche aufgebracht wird, weniger als 12 s beträgt, und dass die Zeit, die benötigt wird für das vollständige Eindringen in die Basiszusammensetzung der gleichen Menge, 20 µl, an Tinte, die an ihrer Oberfläche aufgebracht wird, weniger als 12 s beträgt.

3. Verfahren nach Anspruch 1, wobei der Schritt des Herstellens zweier Drucke einer Tinte oder Tinten so durchgeführt wird, dass der Abstand d zwischen den benachbarten Kanten der Aufbringungen der beiden Zonen kleiner oder gleich der Summe (l₁ + l₂) der seitlichen Diffusionsbreiten jeder der Druckfarben multipliziert mit einem Koeffizienten k zwischen 1 und 2 ist, d.h. d ≤ (l₁ + l₂)*k, wobei die seitliche Diffusionsbreite (l₁, l₂) vorzugsweise kleiner als oder gleich 5 mm, vorzugsweise kleiner als oder gleich 2 mm, vorzugsweise kleiner als oder gleich 1 mm ist.

4. Verfahren nach einem der vorhergehenden Ansprüche, wobei auf die erste Zone eine Tinte einer ersten Farbe und auf die zweite Zone eine Tinte einer zweiten Farbe aufgebracht wird.

5. Verfahren nach einem der vorhergehenden Ansprüche, wobei die Anzahl der Zonen größer als zwei ist, wobei die Anzahl der Farben der durch das Drucken aufgebrachten Tinten gleich zwei ist, wobei die Zonen in einer vorbestimmten Art und Weise zusammen angeordnet sind, insbesondere im Wechsel, insbesondere in Form eines Karomusters, oder die Anzahl der Zonen und der Farben der aufgebrachten Druckfarben größer als zwei ist.

6. Verfahren nach einem der vorhergehenden Ansprüche, wobei jede Zone der Basiszusammensetzung durch ein von der Zusammensetzung verschiedenes Abgrenzungselement abgegrenzt ist, insbesondere jede Zone durch ein Schälchen definiert ist, in dem die Zusammensetzung in dieser Zone untergebracht ist, wobei das Schälchen dieser Zone von dem einer benachbarten Zone getrennt ist oder mindestens zwei Zonen durch ein gleiches Schälchen definiert sind, das die Zusammensetzung enthält.

7. Verfahren nach einem der vorhergehenden Ansprüche, wobei jede Zone eine polygonale oder nicht-polygonale Kontur, insbesondere eine kreisförmige oder nicht kreisförmige, zum Beispiel ovale, Kontur aufweist.

8. Verfahren nach einem der vorhergehenden Ansprüche, wobei der Schritt des Aufbringens von Tinte oder Tinten so durchgeführt wird, dass eine Abstufung der Farben zwischen zwei benachbarten Zonen erzeugt wird.

9. Verfahren nach einem der vorhergehenden Ansprüche, wobei der Schritt des Aufbringens von Tinte oder Tinten so durchgeführt wird, dass eine Vielzahl von Zonen abgegrenzt wird, wobei die Basiszusammensetzung auf mindestens einer Zone eine dritte Verbindung enthält oder dazu bestimmt ist, eine dritte Verbindung zu enthalten, wobei die dritte Verbindung vorzugsweise ein Wirkstoff ist, der aus der Gruppe ausgewählt ist, die aus kosmetischen und/oder dermatologischen Wirkstoffen, wie UV-Schutzmitteln und Anti-Akne-Wirkstoffen besteht, wobei das Verfahren vorzugsweise den Schritt umfasst, der darin besteht, unter Verwendung des Druckers die dritte Verbindung gleichzeitig oder nach dem Schritt des Aufbringens von Tinte oder Tinten aufzubringen.

10. Verfahren nach einem der vorhergehenden Ansprüche, wobei der Schritt des Aufbringens von Tinte oder Tinten darin besteht, eine Anzahl n > 1 von Druckdurchgängen durchzuführen, wobei n vorzugsweise zwischen 5 und 200 liegt.

11. Verfahren nach einem der vorhergehenden Ansprüche, wobei die Basiszusammensetzung vor dem Aufbringen der Drucke der Tinte oder Tinten weiß ist, wobei die Basiszusammensetzung vorzugsweise eine Basis, ein Blusher, ein Lidschatten oder ein Lippenstift ist.

12. Verfahren nach einem der vorhergehenden Ansprüche, wobei das Aufbringen der Tinte oder Tinten über alle Zonen die gesamte Oberfläche der Basiszusammensetzung abdeckt.

13. Verfahren nach einem der vorhergehenden Ansprüche, wobei die Gesamtmenge an Tinte oder Tinten, die auf der Basiszusammensetzung aufgebracht wird, größer oder gleich 0,2 mg/cm² ist.

## Revendications

1. Procédé de coloration d'une composition cosmétique de base, comprenant l'étape consistant à produire, sur au moins une première zone et une deuxième zone, qui sont différentes, à la surface de ladite composition, deux impressions d'au moins une encre provenant d'une imprimante à jet d'encre, la composition de base étant pulvérulente sous forme de poudre compacte, l'au moins une encre étant une encre aqueuse comprenant une concentration en poids d'au moins 50 % d'eau et ayant une viscosité dans la plage de 1 mPa.s à 500 mPa.s à 25 °C, la composition de base et l'encre étant telles que la durée nécessaire pour la pénétration complète, à une température ambiante de 20 °C et sous pression atmosphérique, dans la composition de base d'une quantité de 20 µl d'eau déposée à sa surface est inférieure à 30 s et telles que la durée nécessaire pour la pénétration complète dans la composition de base de la même quantité, 20 µl, d'encre déposée à sa surface est inférieure à 15 s, dans lequel l'encre est déposée en une quantité suffisante de manière à diffuser sur une profondeur de la composition de base supérieure ou égale à 1 mm, pour chaque zone.

2. Procédé selon la revendication 1, dans lequel la composition de base et l'encre sont telles que la durée nécessaire pour la pénétration complète dans la composition de base d'une quantité de 20 µl d'eau déposée à sa surface est inférieure à 12 s et telles que la durée nécessaire pour la pénétration complète dans la composition de base de la même quantité, 20 µl, d'encre déposée à sa surface est inférieure à 12 s.

3. Procédé selon la revendication 1, l'étape de production des deux impressions d'encre(s) étant conduite de manière à ce que la distance d entre les bords adjacents des dépôts des deux zones soit inférieure ou égale à la somme (l₁ + l₂) des largeurs de diffusion latérale de chacune des encres multipliée par un coefficient k compris entre 1 et 2 soit d ≤ (l₁+l₂)*k, la largeur (l₁, l₂) de diffusion latérale étant de préférence inférieure ou égale à 5 mm, de préférence inférieure ou égale à 2 mm, de préférence inférieure ou égale à 1 mm.

4. Procédé selon l'une quelconque des revendications précédentes, une encre d'une première couleur étant déposée sur la première zone et une encre d'une deuxième couleur étant déposée sur la deuxième zone.

5. Procédé selon l'une quelconque des revendications précédentes, le nombre de zones étant supérieur à deux, le nombre de couleurs d'encres déposées par impression étant égal à deux, les zones étant disposées entre elles de manière prédéterminée, notamment en alternance, en particulier sous forme de damier, ou le nombre de zones et de couleurs d'encres déposées étant supérieur à deux.

6. Procédé selon l'une quelconque des revendications précédentes, chaque zone de la composition de base étant délimitée par un élément de démarcation différent de la composition, notamment chaque zone étant définie par une coupelle dans laquelle la composition dans cette zone est logée, la coupelle de cette zone étant distincte de celle d'une zone adjacente, ou au moins deux zones étant définies par une même coupelle contenant la composition.

7. Procédé selon l'une quelconque des revendications précédentes, chaque zone ayant un contour polygonal ou non polygonal, notamment circulaire ou non circulaire, par exemple ovale.

8. Procédé selon l'une quelconque des revendications précédentes, l'étape de dépôt des impressions d'encre(s) étant effectuée de manière à réaliser un dégradé de couleurs entre deux zones adjacentes.

9. Procédé selon l'une quelconque des revendications précédentes, l'étape de dépôt des impressions d'encre(s) étant effectuée de manière à délimiter une pluralité de zones, la composition de base sur au moins une zone contenant ou étant destinée à contenir un composé tiers, le composé tiers étant de préférence un agent actif choisi dans le groupe constitué d'agents actifs cosmétiques et/ou dermatologiques, tels que des filtres UV et des agents actifs anti-acné, le procédé comprenant de préférence l'étape consistant à déposer, à l'aide de l'imprimante, ledit composé tiers, simultanément ou après l'étape de dépôt des impressions d'encre(s).

10. Procédé selon l'une quelconque des revendications précédentes, l'étape de dépôt des impressions d'encre(s) consistant à effectuer un nombre *n* > 1 de passes d'impression, n étant de préférence compris entre 5 et 200.

11. Procédé selon l'une quelconque des revendications précédentes, la composition de base étant blanche avant dépôt des impressions d'encre(s), la composition de base étant de préférence un fond de teint, un blush, un fard à paupières ou un rouge à lèvres.

12. Procédé selon l'une quelconque des revendications précédentes, le dépôt d'encre(s) sur l'ensemble des zones couvrant l'ensemble de la surface de la composition de base.

13. Procédé selon l'une quelconque des revendications précédentes, la quantité totale d'encre(s) déposée sur la composition de base étant supérieure ou égale à 0,2 mg/cm².
